(19) Europäisches Patentamt
European Patent Office
Office européen des brevets

(11) Veröffentlichungsnummer: **0 005 153**
**B1**

(12) # EUROPÄISCHE PATENTSCHRIFT

(45) Veröffentlichungstag der Patentschrift: **27.01.82**

(51) Int. Cl.³: **C 07 C 149/32,** A 01 N 41/12, C 07 D 521/00

(21) Anmeldenummer: **79100642.2**

(22) Anmeldetag: **05.03.79**

(54) **4-Nitro-2-trichlormethylphenyldisulfide, Verfahren zu ihrer Herstellung, den Wirkstoff enthaltende Mittel und Verwendung gegen Mikroorganismen.**

(30) Priorität: **11.03.78 DE 2810698**

(43) Veröffentlichungstag der Anmeldung: **14.11.79 Patentblatt 79/23**

(45) Bekanntmachung des Hinweises auf die Patenterteilung: **27.01.82 Patentblatt 82/4**

(84) Benannte Vertragsstaaten: **BE CH DE FR GB IT NL SE**

(56) Entgegenhaltungen:
**US-A-2 962 417**
**CHEMICAL ABSTRACTS, Vol. 81, Nr. 21, 25. November 1974, Zusammenfassung Nr. 135720c, Columbus, Ohio, USA, TAKAHASHI SABURO et al.: »Asymmetric disulfides«, Seite 389, rechte Spalte**
**CHEMICAL ABSTRACTS, Vol. 81, Nr. 5, 5. August 1974, Zusammenfassung Nr. 22237g, Columbus, Ohio, USA, TAKAHASHI SABURO et al.: »Agricultural pesticides and herbicides containing disulfide derivatives«, Seite 154, rechte Spalte**

(73) Patentinhaber: **BASF Aktiengesellschaft, Carl-Bosch-Strasse 38, D-6700 Ludwigshafen (DE)**

(72) Erfinder: **Hagen, Helmut, Dr., Max-Slevogt-Strasse 17 e, D-6710 Frankenthal (DE)**
Erfinder: **Reuther, Wolfgang, Dr., Am Pferchelhang 16, D-6900 Heidelberg-Ziegelhausen (DE)**
Erfinder: **Pommer, Ernst-Heinrich, Dr., Berliner Platz 7, D-6703 Limburgerhof (DE)**

**EP 0 005 153 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 4-Nitro-2-trichlormethylphenyldisulfide, Verfahren zu ihrer Herstellung den Wirkstoff enthaltende Mittel und Verwendung gegen Mikroorganismen

Die vorliegende Erfindung betrifft neue, wertvolle 4-Nitro-2-trichlormethylphenyldisulfide mit mikrozider, fungizider und bakterizider Wirkung, Verfahren zu ihrer Herstellung, Mikrozide, die Verwendung dieser Verbindungen als Wirkstoffe zur Bekämpfung von Pilzen und Bakterien.

Es ist beispielsweise bekannt, N-Trichlormethylthio-phthalimid (Chemical Week 1972, June 21, Seite 63), Tetramethylthiuramdisulfide (Chemical Week 1972, July 26, Seite 39) oder 2-Thiocyanomethylthio-benzothiazol (Farm Chemicals Handbook 1976, Seite D 43) als Fungizide zu verwenden; ferner sind die Trifluormethylgruppe enthaltende Disulfide aus Chem. Abstr., 81, 1974, 22 237 g bekannt geworden. Ihre Wirkung ist jedoch unbefriedigend.

Aufgabe der vorliegenden Erfindung ist daher die Entwicklung neuer Wirkstoffe und Mikrozide mit besserer Wirkung.

Es wurde gefunden, daß 4-Nitro-2-trichlormethylphenyldisulfide der Formel 1

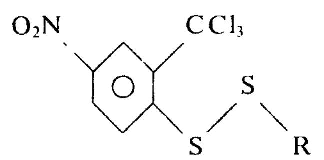

(1)

in der R einen gegebenenfalls substituierten aliphatischen Rest mit 1 bis 16 Kohlenstoffatomen, einen gegebenenfalls substituierten araliphatischen Rest mit insgesamt 7 bis 9 Kohlenstoffatomen, einen gegebenenfalls substituierten ein- oder mehrkernigen aromatischen Rest oder einen gegebenenfalls substituierten 1 bis 3 Heteroatome enthaltenden 5- oder 6gliedrigen Ring mit Sauerstoff, Stickstoff oder Schwefel als Heteroatome oder dessen Benzhomologes den 1,2,3,4-Tetrazolyl- oder den 1-Phenyl-(1,2,3,4-tetrazolyl)-rest bedeutet, eine bessere fungizide Wirkung als die oben genannten bekannten Wirkstoffe haben.

Als aliphatische Reste mit 1 bis 16 Kohlenstoffatomen kommen geradkettige oder verzweigte Alkylreste, gegebenenfalls ein- oder mehrfach substituiert durch Carboxy-, Hydroxy-, Nitril-, Amino-, Dialkylamino-, Alkoxy- oder Carbalkoxygruppen mit jeweils 1 bis 3 C-Atomen im Alkyl oder Ketogruppen, wie Methyl, Äthyl, n- und i-Propyl, n-Butyl, n-Hexyl, i-Octyl, n-Dodecyl, n-Tridecyl, Carboxymethyl, Methoxyäthyl, Dimethylaminoäthyl, Aminoäthyl, L-Alanyl, Carboxyäthyl, Acetyl, Carbomethoxymethyl, Hydroxyäthyl oder Dicarboxyäthyl, in Betracht.

Als araliphatische Reste kommen beispielsweise Benzyl oder Phenyläthyl, die im Phenyl gegebenenfalls 1- bis 3fach durch Chloratome, Nitrogruppen und/oder Cyangruppen substituiert sein können, wie Dichlorbenzyl, Nitrobenzyl, in Betracht.

Als aromatische Reste kommen gegebenenfalls 1- bis 3fach substituierte Benzolkohlenwasserstoffe, insbesondere Phenyl- und Naphthalinreste, die durch Cyangruppen, Halogenatome, insbesondere Chloratome, und/oder Nitrogruppen substituiert sein können, in Betracht, von denen beispielsweise Cyanchlorphenyl, Cyannaphthyl, Cyannitrophenyl, Chlornitrophenyl, Cyandichlorphenyl genannt seien. Die genannten Phenylreste können zusätzlich einen heterocyclischen Ring als Substituenten aufweisen, wie einen Imidazolin-, einen Methylimidazolin- oder einen Tetrahydropyrimidinrest.

Als heterocyclische Reste kommen für R der Benzthiazolrest, Benzimidazolrest, Thiazolrest, Imidazolrest, Thiadiazolrest, Oxazolrest, Benzoxazolrest, Oxdiazolrest oder Pyridylrest, die im Benzolring 1- bis 3fach durch Chloratome, Nitrogruppen und/oder Cyangruppen und/oder im heterocyclischen Ring durch Phenyl substituiert sein können, in Betracht. Weitere heterocyclische Reste sind der Thiazolinrest, der Imidazolinrest, der 1,2,3,4-Tetrazolrest oder der Pyridinrest. Einige der 5-Ringglieder enthaltenden Heterocyclen entsprechen der Formel 1, in der

R den Rest

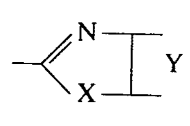

X = O, S oder NH
Y = 2 Wasserstoffatome, eine Doppelbindung oder einen anellierten Phenylrest

bedeuten.

Von den für R genannten Bedeutungen sind bevorzugt Alkylreste mit 2 bis 12 C-Atomen, gegebenenfalls ein- oder mehrfach durch Carboxy-, Carbalkoxy-, Hydroxy-, Amino- oder eine Ketogruppe substituiert, Phenyl oder Naphthyl, gegebenenfalls 1- bis 2fach durch Chloratome, Cyan- und/oder Nitrogruppen und/oder einen heterocyclischen Rest substituiert, sowie Thiazol oder Imidazolin, gegebenenfalls durch Phenyl substituiert, und Benzthiazol, Benzimidazol und Benzoxazol, jeweils gegebenenfalls durch ein Chloratom substituiert.
Von den bevorzugten Bedeutungen seien beispielsweise genannt;
n-Butyl, i-Butyl, n-Dodecyl, Tridecyl-, Carboxymethyl, Phenyl, Chlorphenyl, Dichlorphenyl, Chlorcyanphenyl, Nitrophenyl, Chlornitrophenyl, Cyannaphthyl, Benzthiazolyl, Chlorbenzthiazolyl, Benzimidazolyl, Thiazolyl, Benzoxazolyl, 2-Imidazolinyl-phenyl.

Als besonders bevorzugte Verbindung ist 2-(4-Nitro-2-trichlormethylphenyl-dithio)-1,3-benzthiazol hervorzuheben. Sie eignet sich insbesondere als Mittel zum Schutz von Holz gegen Pilzbefall und kann dazu auf vorteilhafte Weise in Lösemittel enthaltenden Formulierungen angewendet werden.

Die neuen Wirkstoffe eignen sich insbesondere zum Schutz von verschiedenen Materialien gegen den Abbau bzw. die Zerstörung durch Bakterien und Pilze. Materialien, die mit den neuen Wirkstoffen konserviert bzw. mikrozid ausgerüstet werden können, sind beispielsweise Leime und Klebstoffe, Kunststoffdispersionen, Dispersionsfarben, Dichtungsmassen, Papier, Textilien, Leder, Rohhäute, Kunststoffe, insbesondere Weich-PVC, Gummi und Holz. Ferner sind die Verbindungen als mikrozid wirksame Zusätze in Reinigungs- und Desinfektionsmitteln sowie als Schleimbekämpfungsmittel in der Papierindustrie geeignet.

Folgende Mikroorganismen lassen sich beispielsweise mit den erfindungsgemäßen Verbindungen zum Schutz von Material bekämpfen:
Chaetomium globosum, Chaetomium alba, Aspergillus terreus, Aspergillus niger, Aspergillus versicolor, Penicillium glaucum, Penicillium funiculosum, Trichoderma viride, Pullularia pullulans, Cladosporium herbarum, Cladosporium resinae, Humicola grisea, Glenospora graphii, Phoma violacea; Coniophora cerebella, Merulius lacrymans, Poria monticola, Lenzites trabea, Lenzites abietina, Trametes versicolor, Armillaria mellea; Streptomyces albus; Staphylococcus aureus, Escherichia coli, Pseudomonas aeruginosa, Aerobacter aerogenes, Serratia marcescens.

Die Mittel, welche die 4-Nitro-2-trichlormethylphenyldisulfide als Wirkstoffe enthalten, eignen sich, allgemein gesagt, zum Schutz vieler Substrate gegen den Befall durch Mikroorganismen und zur Bekämpfung von Mikroorganismen auf oder in den von ihnen befallenen Substraten.

Ein bevorzugtes Anwendungsgebiet ist der Holzschutz, das heißt der Schutz von Holz und Holzwerkstoffen z. B. Spanplatten gegen den Befall durch Mikroorganismen und die Bekämpfung von Mikroorganismen, welche diese Substrate befallen haben.

Die Herstellung der erfindungsgemäßen Verbindungen erfolgt zweckmäßigerweise durch Umsetzung von 4-Nitro-2-trichlormethyl-benzolsulfensäurechlorid der Formel 2 mit einer eine Mercaptogruppe enthaltende Verbindung der Formel 3, in der R die für Formel 1 oben angegebenen Bedeutungen hat, in einem inerten Lösungsmittel bei Temperaturen von −40 bis 120°C.

Die Umsetzung läßt sich durch folgende Reaktionsgleichung beschreiben:

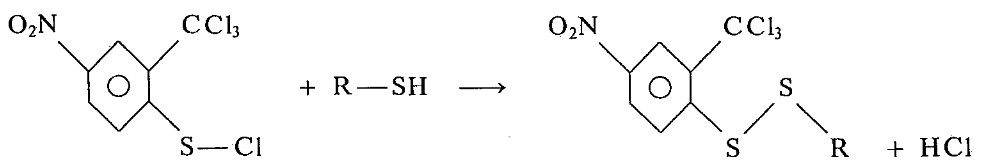

2 3

Für die Umsetzungen kommen als inerte Lösungsmittel zweckmäßigerweise gesättigte aliphatische oder cyclische Äther, wie Diäthyläther, Tetrahydrofuran, Dioxan, Dibutyläther, aliphatische Carbonsäureester, wie Essigsäureäthylester, Essigsäurebutylester, niedere Alkohole, wie Isobutanol, chlorierte Kohlenwasserstoffe, wie Methylenchlorid, aromatische Kohlenwasserstoffe, wie Benzol, Toluol oder Xylol, in Betracht.

Von den genannten Lösungsmitteln sind insbesondere bevorzugt Diäthyläther, Tetrahydrofuran, Dibutyläther, Essigsäureäthylester, Isobutanol und Toluol.

Der für die Umsetzung bevorzugte Temperaturbereich liegt bei Temperaturen von 0 bis +60°C. Verbindungen, die eine basische Gruppe im Rest R enthalten, bilden bei der erfindungsgemäßen Herstellung das Hydrochlorid und können ohne Schwierigkeiten als freie Base gewonnen werden.

Das als Ausgangsverbindung verwendete 4-Nitro-2-trichlormethylbenzolsulfensäurechlorid wird zweckmäßigerweise nach dem in der deutschen Patentanmeldung P 27 21 917.6 beschriebenen Verfahren durch Chlorierung von 5-Nitrobenzo-1,2-dithio-3-thion in einem inerten Lösungsmittel, wie Tetrachlorkohlenstoff, Chloroform oder Dichloräthan, bei Temperaturen von −20 bis 100°C, vorzugsweise von 0 bis 50°C, hergestellt.

Die folgende Vorschrift erläutert dieses Verfahren:

115 g 5-Nitrobenzo-1,2-dithio-3-thion werden in 1000 ml Tetrachlorkohlenstoff dispergiert und bei 10°C 150 g eingeleitet. Das Reaktionsgemisch wird noch 12 Stunden bei Raumtemperatur (25°C) gerührt. Das Lösungsmittel und Schwefelchloride werden danach im Vakuum einer Wasserstrahlpumpe abdestilliert. Der Rückstand kann ohne weitere Reinigung zur Synthese der erfindungsgemäßen Disulfide verwendet werden. Er kann aber auch mit 400 ml Diäthyläther versetzt werden, die Ätherlösung wird filtriert und anschließend eingeengt und im Vakuum destilliert. Bei $Kp_1 = 156$ bis 160°C wird das 4-Nitro-2-trichlormethyl-benzolsulfensäurechlorid erhalten. Die Verbindung hat einen Schmelzpunkt von 56 bis 57°C (aus Ligroin).

Die neuen Wirkstoffe werden angewendet in Form von Zubereitungen. Gegenstand der vorliegenden Erfindung sind demnach auch Mittel oder Zubereitungen, die neben üblichen Trägern und Verdünnungsmitteln eine Verbindung der Formel 1 enthalten.

Die Zubereitungen, wie Lösungen, Emulsionen, Suspensionen, benetzbare Pulver, Stäube, Pasten oder Granulate werden in bekannter Weise angewendet, beispielsweise Mischen, Anstreichen, Tränken, Beizen. Die Zubereitungen enthalten im allgemeinen zwischen 0,1 und 95 Gew.-% Wirkstoff, vorzugsweise 0,5 bis 90%. Die Aufwandmengen betragen je nach Art des gewünschten Effektes 0,001 bis 5% (Gewichtsprozent) bezogen auf das Gewicht des zu schützenden Materials, vorzugsweise jedoch zwischen 0,01 und 3%.

Zur Herstellung von Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Napthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenol, Alkylphenolpolyglykoläther, Tributylphenolpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Die folgende Liste von Bakteriziden und Fungiziden, mit denen die erfindungsgemäßen Verbindungen kombiniert werden können, soll die Mischungsmöglichkeiten aufzeigen, nicht aber einschränken. Durch die Kombination mit anderen Wirkstoffen erhält man in vielen Fällen eine Vergrößerung des mikroziden Wirkungsspektrums:

Organozinnverbindungen, wie Tributylzinnoxid und Tributylzinnbenzoat
2-Brom-2-nitro-1,3-propandiol
Methylenbisthiocyanat
Chloräthylenbisthiocyanat
Formaldehyd
Glutaraldehyd
Chloracetamid
N-Methylol-chloracetamid
Natrium- und Zinkdimethyldithiocarbamat
Tetramethylthiuramdisulfid
1,6-Bis-(4-chlorphenyl-diguanido)-hexan
Alkyl-trimethyl-ammoniumchlorid
Alkyl-dimethyl-benzylammoniumchlorid
Cetyl-pyridiniumchlorid
Dodecyl-di-(aminoäthyl)-glycin

o-Phenol-phenol
p-Chlor-m-kresol
Chlorierte Phenole
p-Hydroxybenzoesäureester
Tetrachlorisophthalsäure-dinitril
Halogenierte Salicylanilide
2-Halogenbenzoesäureanilid
N-Cyclohexyl-N-methoxy-2,5-dimethyl-furan-3-carbonsäureamid
N,N-Dimethyl-N'-phenyl-(N-fluordichlormethylthio)-sulfamid
N-Phenyl-N,N'-dimethyl-N'-fluordichlormethylthio-sulfonyldiamid
N-Trichlormethylthiophthalimid
N-(1,1,2,2-Tetrachloräthylthio)-tetrahydrophthalimid
Benzimidazol-2-carbaminsäure-methylester
2-(Thiazolyl)-benzimidazol
2-Mercaptobenzthiazol
2-Thiocyanomethyl-thiobenzothiazol
Benzisothiazolon
2,5-Dimethyl-tetrahydro-1,3,5-2H-thiadiazin-2-thion
Alkali- und Metallsalze des N'-Hydroxy-N-cyclohexyl-diazeniumoxids.

Die Zumischung dieser Wirkstoffe zu den erfindungsgemäßen Verbindungen kann im Gewichtsverhältnis 1 : 10 bis 10 : 1 erfolgen.

Die erfindungsgemäßen Wirkstoffe haben auch eine starke fungitoxische Wirkung gegenüber phytopathogenen Pilzen, insbesondere aus den Pilzklassen der Phycomyceten und Ascomyceten.

Die neuen Verbindungen sind daher beispielsweise geeignet zur Bekämpfung von Plasmopara viticola an Reben, Pseudoperonospora humuli an Hopfen, Phytophthora infestans an Kartoffeln und Tomaten, Septoria nodorum an Getreide und Venturia inaequalis an Apfelbäumen. Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 und 3 kg Wirkstoff je ha.

Die Herstellung der neuen Wirkstoffe wird in den folgenden Beispielen erläutert.

## Beispiel 1

Butyldithio-4-nitro-2-trichlormethylbenzol

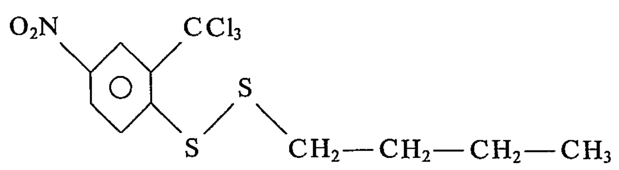

In der Rührapparatur werden 61 g 4-Nitro-2-trichlormethylbenzolsulfensäurechlorid in 800 ml Diäthyläther mit 18 g 1-Butylmercaptan bei 25°C 6 Stunden gerührt. Das Ende der Umsetzung wird gaschromatographisch bestimmt. Nach dem Entfernen des Lösungsmittels erhält man 66 g (4-Nitro-2-trichlormethylphenyl)-n-butyldisulfid als gelbes Öl.

Ausbeute: 92% d. Th.

Elementaranalyse:

| | | | | | | |
|---|---|---|---|---|---|---|
| ber. | C 36.2 | H 3.2 | O 9.3 | N 4.1 | S 17.2 | Cl 29.7 |
| gef. | C 36.6 | H 3.3 | O 8.9 | N 3.9 | S 17.8 | Cl 29.5 |

## Beispiel 2

### i-Propyldithio-4-nitro-2-trichlormethylbenzol

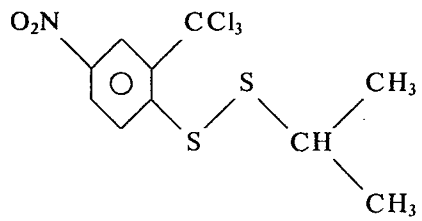

Analog Beispiel 1 werden 61 g 4-Nitro-2-trichlormethylbenzolsulfensäurechlorid mit 15 g 2-Propylmercaptan umgesetzt. Man erhält 63 g (4-Nitro-2-trichlormethylphenyl)-isopropyldisulfid.

Ausbeute: 91% d. Th.

Analyse:

| | | | | | | |
|---|---|---|---|---|---|---|
| ber. | C 34.2 | H 2.8 | O 9.5 | N 4.1 | S 18.2 | Cl 30.5 |
| gef. | C 34.6 | H 2.9 | O 9.3 | N 4.1 | S 18.4 | Cl 30.7 |

## Beispiel 3

### n-Propyldithio-4-nitro-2-trichlormethylbenzol

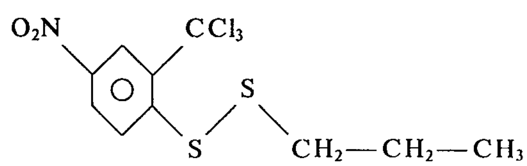

Analog werden 61 g 4-Nitro-2-trichlormethylbenzolsulfonsäurechlorid mit 15 g 1-Propylmercaptan umgesetzt. Man erhält 62 g (4-Nitro-2-trichlormethylphenyl)-n-propyldisulfid als gelbes Öl.

Ausbeute: 89% d. Th.

Analyse:

| | | | | | | |
|---|---|---|---|---|---|---|
| gef. | C 34.3 | H 2.8 | O 9.6 | N 4.1 | S 18.3 | Cl 30.6 |
| ber. | C 34.6 | H 2.9 | O 9.3 | N 4.1 | S 18.4 | Cl 30.7 |

## Beispiel 4

### 3-Chlor-2-cyanphenyldithio-4-nitro-2-trichlormethylbenzol

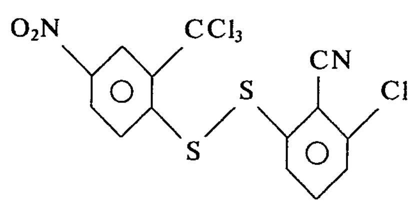

In einer Rührapparatur werden 30,7 g 4-Nitro-2-trichlormethylbenzolsulfensäurechlorid in 500 ml Diäthyläther mit 17 g 2-Cyan-3-chlorthiophenol 4 Stunden bei 30°C umgesetzt. Man erhält 38 g (4-Nitro-2-trichlormethylphenyl)-(2-cyan-3-chlorphenyl)-disulfid mit Fp. = 137°C.

Ausbeute: 86% d. Th.

Analyse:

| | | | | | | |
|---|---|---|---|---|---|---|
| gef. | C 38.4 | H 1.4 | O 7.3 | N 6.4 | S 14.7 | Cl 32.2 |
| ber. | C 38.2 | H 1.4 | O 7.3 | N 6.4 | S 14.6 | Cl 32.3 |

Beispiel 5

n-Dodecyldithio-4-nitro-2-trichlormethylbenzol

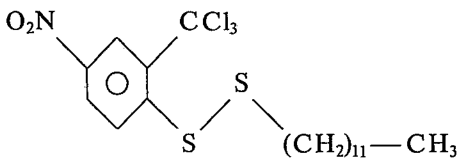

Analog Beispiel 1 werden 61,4 g 4-Nitro-2-trichlormethylbenzolsulfensäurechlorid mit 40,5 g Dodecylmercaptan umgesetzt. Man erhält 81 g (4-Nitro-2-trichlormethylphenyl)-dodecyldisulfid.

Ausbeute: 86% d. Th.

Analyse:

| | | | | | | |
|---|---|---|---|---|---|---|
| gef. | C 48.8 | H 6.2 | O 6.9 | N 2.7 | S 13.4 | Cl 21.8 |
| ber. | C 48.3 | H 6.0 | O 6.8 | N 2.9 | S 13.5 | Cl 22.5 |

Beispiel 6

Carboxymethyldithio-4-nitro-2-trichlormethylbenzol

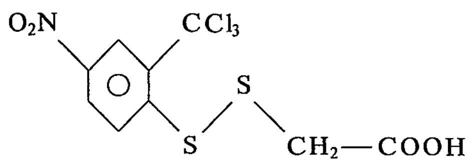

Analog Beispiel 1 werden 61,4 g 4-Nitro-2-trichlormethylbenzolsulfensäurechlorid mit 18,4 g Thioglykolsäure umgesetzt. Nach dem Abdestillieren des Lösungsmittels und Umkristallisieren aus Toluol erhält man 62 g Carboxymethyldithio-4-nitro-2-trichlormethylbenzol mit Fp. = 140° C.

Analyse:

| | | | | | | |
|---|---|---|---|---|---|---|
| gef. | C 30.0 | H 1.8 | O 17.6 | N 3.9 | S 17.3 | Cl 29.3 |
| ber. | C 29.8 | H 1.7 | O 17.6 | N 3.9 | S 17.6 | Cl 29.3 |

Beispiel 7

2-(4-Nitro-2-trichlormethylphenyldithio)-1,3-benzthiazol

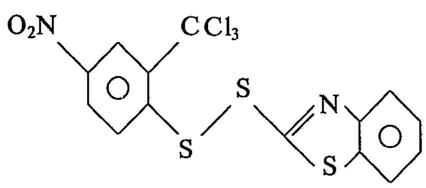

61,4 g 4-Nitro-2-dichlormethylbenzolsulfensäurechlorid werden in 800 ml Isobutanol bei 25 bis 30° C mit 33,5 g 2-Mercaptobenzthiazol umgesetzt. Man erhält 84 g 2-(4-Nitro-2-trichlormethylphenyldithio)-1,3-benzthiazol mit Fp. = 97° C. Die Ausbeute entspricht 96% d. Th.

Analyse:

| | | | | | | |
|---|---|---|---|---|---|---|
| gef. | C 38.8 | H 1.7 | O 7.2 | N 6.4 | S 21.7 | Cl 24.0 |
| ber. | C 38.4 | H 1.6 | O 7.3 | N 6.4 | S 21.9 | Cl 24.3 |

Beispiel 8

Methyldithio-4-nitro-2-trichlormethylbenzol

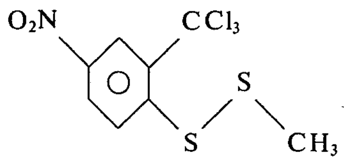

Zu einer Lösung von 46 g 4-Nitro-2-trichlormethylbenzolsulfensäurechlorid in 750 ml Diäthyläther werden bei 5°C 7,2 g Methylmercaptan in 20 Minuten eingeleitet. Man hält das Reaktionsgemisch noch 2 Stunden bei 5 bis 10°C. Nach dem Abdestillieren des Lösungsmittels wird der Rückstand aus Ligroin umkristallisiert. Man erhält 42 g Methyldithio-4-nitro-2-trichlormethylbenzol mit Fp.=76°C. Die Ausbeute entspricht 88% d. Th.

Analyse:

| | | | | | | |
|---|---|---|---|---|---|---|
| gef. | C 30.2 | H 2.1 | O 10.2 | N 4.4 | S 19.9 | Cl 33.2 |
| ber. | C 30.1 | H 1.9 | O 10.0 | N 4.4 | S 20.1 | Cl 33.4 |

Beispiel 9

2-(4-Nitro-2-trichlormethylphenyl-dithio-)-1-cyannaphthalin

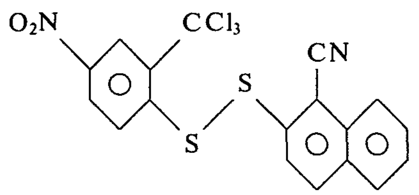

30,7 g 4-Nitro-2-trichlormethylbenzolsulfensäurechlorid werden in 500 ml Dioxan mit 18,5 g 1-Cyan-2-mercapto-naphthalin umgesetzt. Man erhält 37 g 2-(4-Nitro-2-triohlormethylphenyl-dithio-)-1-cyan-naphthalin mit Fp.=170°C (aus Toluol). Die Ausbeute entspricht 81% d. Th.

Analyse:

| | | | | | | |
|---|---|---|---|---|---|---|
| gef. | C 47.2 | H 2.0 | O 7.6 | N 6.2 | S 13.7 | Cl 23.2 |
| ber. | C 47.0 | H 1.9 | O 7.8 | N 6.1 | S 13.9 | Cl 23.2 |

Gemäß Beispiel 1 werden die folgenden Verbindungen hergestellt:

Beispiel 10

(4-Chlor-2-(2)-imidazolinylphenyl)-dithio-2-trichlormethyl-4-nitrobenzol

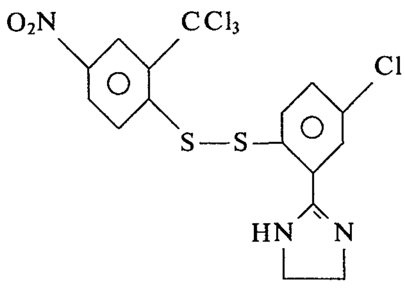

Schmp.: 260° Zers.

Beispiel 11

2-(1,3-Benzimidazol)-dithio-4-nitro-2-trichlormethylbenzol

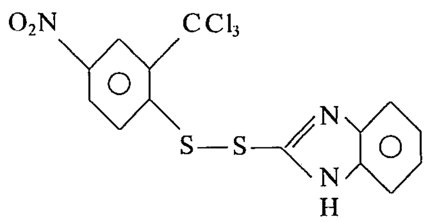

Schmp.: 170°

Beispiel 12

[2-(5-Methylimidazolin)-4-nitro-phenyl-]-dithio-4-nitro-2-trichlormethylbenzolhydrochlorid

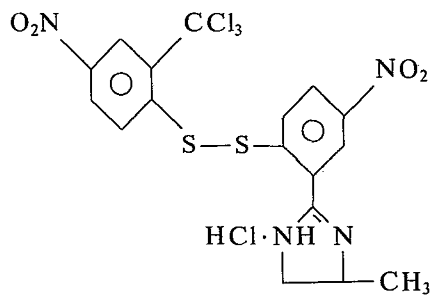

Schmp.: 160°

Beispiel 13

[2-(Tetrahydropyrimidinyl-)-4-chlor-phenyl]-dithio-4-nitro-2-trichlormethylbenzol

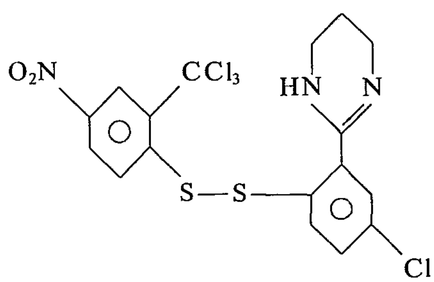

Schm.: 170°

Beispiel 14

1-Phenyl-(1,2,3,4-tetrazol)-dithio-4-nitro-2-trichlormethylbenzol

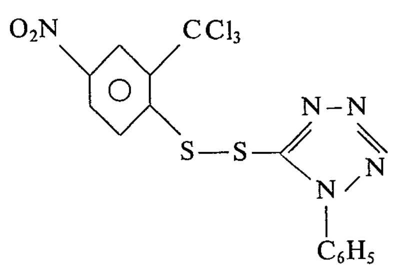

Schmp.: 155°

Beispiel 15

1-Acetyl-dithio-4-nitro-2-trichlormethylbenzol

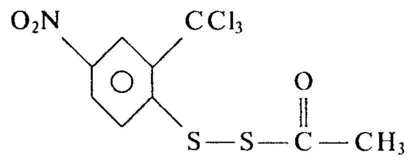

Schmp.: 95°

Beispiel 16

2-(1,3-Thiazolin)-dithio-4-nitro-2-trichlormethylbenzol

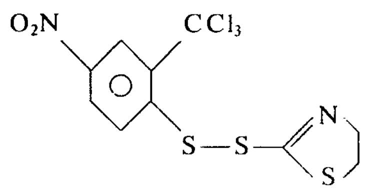

Schmp.: 100°

Beispiel 17

2-Aminoäthyl-dithio-4-nitro-2-trichlormethylbenzolhydrochlorid

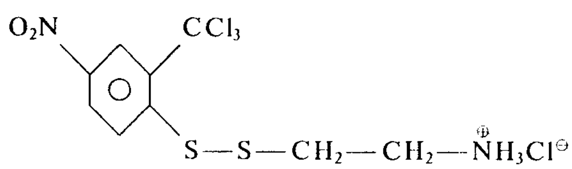

Schmp.: 108°

Beispiel 18

2-(1,3-Imidazolin)-dithio-4-nitro-2-trichlormethylbenzolhydrochlorid

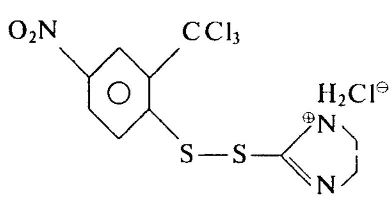

Schmp.: 250°C

Beispiel 19

3′-L-Alanin-dithio-4-nitro-2-trichlormethylbenzol

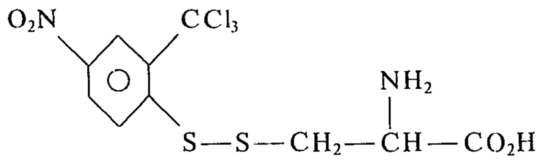

Schmp.: 175°

Beispiel 20

2-Pyridinyl-dithio-4-nitro-2-trichlormethylbenzol

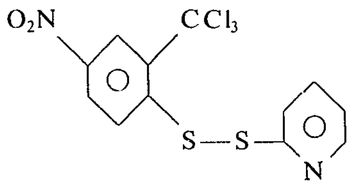

Schmp.: Öl

Beispiel 21

2-Carboxyäthyl-dithio-4-nitro-2-trichlormethylbenzol

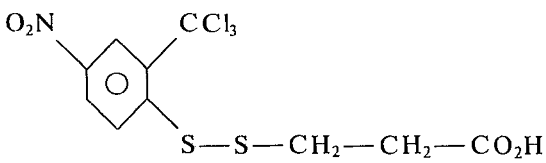

Schmp.: Öl

Beispiel 22

Carbomethoximethyl-dithio-4-nitro-2-trichlormethylbenzol

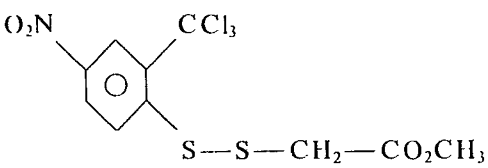

Schmp.: Öl

Beispiel 23

2-Hydroxiäthyl-dithio-4-nitro-2-trichlormethylbenzol

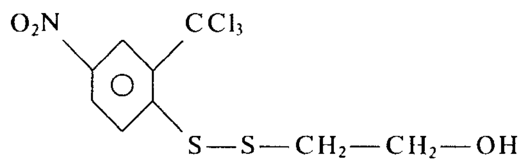

Schmp.: 95°

Beispiel 24

1-(1,2-Dicarboxyäthyl-)dithio-4-nitro-2-trichlormethylbenzol

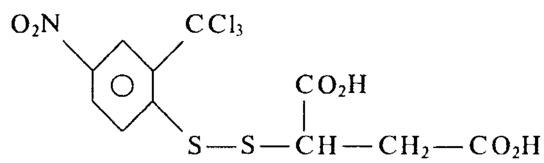

Schmp.: 230°

Beispiel 25

2-(Imidazolin-2-yl)-phenyl-dithio-2-trichlormethyl-4-nitro-benzyl-hydrochlorid

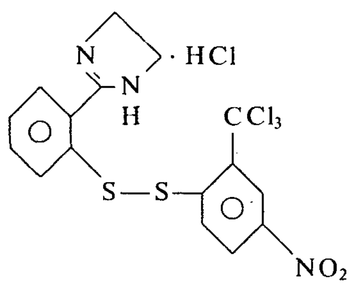

Schmp.: 120–180° (Zers.)

Die Verbindung kristallisiert mit 2 Mol $CH_3OH$.

In den folgenden Beispielen werden Wirksamkeiten von erfindungsgemäßen Verbindungen dargelegt und mit den folgenden drei bekannten Mitteln verglichen:

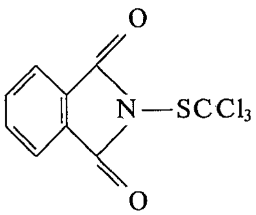

Wirkstoff 11

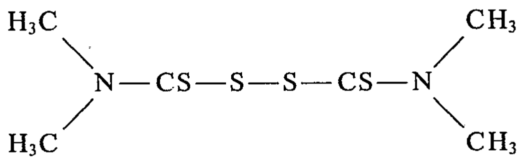

Wirkstoff 12

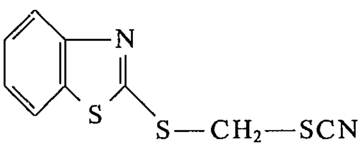

Wirkstoff 13

Beispiel 26

Fungizide Wirksamkeit gegenüber Aspergillus niger

Die Wirkstoffe werden einer für das Wachstum des Pilzes Aspergillus niger optimal geeigneten Nährlösung in Mengen von 100, 50, 25, 10, 5 und 1 Gewichtsteilen pro Million Teile Nährlösung zugesetzt. Es werden jeweils 20 ml der so behandelten Nährlösung in 100 ml Glaskolben mit 0,3 mg Aspergillus-Pilzsporen beimpft. Die Kolben werden 120 Stunden lang bei 36°C erwärmt und anschließend das Ausmaß der Pilzentwicklung, das bevorzugt auf der Nährlösungsoberfläche erfolgt, beurteilt.

0 kein Pilzwachstum, abgestuft bis 5 ungehemmtes Pilzwachstum (Pilzdecke auf der Nährlösungsoberfläche geschlossen).

| Wirkstoff Nr. | 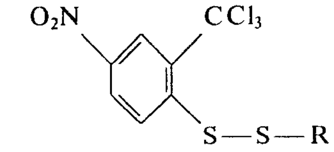 R= | Wirkstoffmenge in der Nährlösung ... Teile pro Million Teile Nährlösung 100 | 50 | 25 | 10 | 5 | 1 |
|---|---|---|---|---|---|---|---|
| 1 | $—CH_2—CH_3$ | 0 | 0 | 0 | 3 | 5 | 5 |
| 2 | $—CH_2—CH_2—CH_3$ | 0 | 0 | 0 | 3 | 5 | 5 |
| 3 | $—CH(CH_3)_2$ | 0 | 0 | 1 | 1 | 4 | 5 |
| 4 | $—CH_2—CH_2—CH_2—CH_3$ | 0 | 0 | 1 | 1 | 4 | 5 |
| 5 | $—(CH_2)_{11}CH_3$ | 0 | 0 | 1 | 3 | 5 | 5 |
| 6 | $—CH_2—COOH$ | 0 | 0 | 1 | 3 | 5 | 5 |
| 7 | 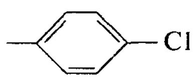 | 0 | 0 | 0 | 1 | 3 | 5 |
| 8 | 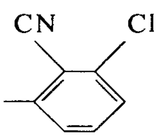 | 0 | 0 | 0 | 0 | 0 | 1 |
| 9 | 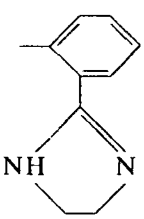 $\cdot HCl \cdot 2CH_3OH$ | 0 | 0 | 0 | 1 | 3 | 5 |
| 10 | 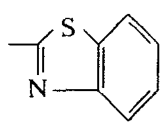 | 0 | 0 | 0 | 1 | 3 | 5 |
| 11 | bekannt | 0 | 1 | 1 | 3 | 5 | 5 |
| 12 | bekannt | 1 | 2 | 4 | 5 | 5 | 5 |
| 13 | bekannt | 0 | 3 | 3 | 5 | 5 | 5 |

Beispiel 27

Zur Herstellung eines öligen Holzschutzmittels mit 2% 2-(4-Nitro-2-trichlormethylphenyl-dithio)-1,3-benzthiazol (Wirkstoff 10) werden 2 Teile des Wirkstoffes mit 15 Teilen eines Alkydharzes mit mittlerem Gehalt an Ölen (20% Festharz) gemischt. Anschließend fügt man 45 Teile einer aromahaltigen Benzinfraktion hinzu, filtriert gegebenenfalls, um Verunreinigungen zu beseitigen, und füllt mit einer aliphatenhaltigen Benzinfraktion auf 100 Teile auf.

Beispiel 28

In analoger Weise werden 2%ige ölige Holzschutzmittel mit den Wirkstoffen 2 und 8 hergestellt.

Beispiel 29

Zur Ermittlung der Wirksamkeit der in den Beispielen 27 und 28 beschriebenen öligen Holzschutzmittel gegenüber den holzzerstörenden Pilzen Coniophora cerebella und Trametes versicolor wurden in Anlehnung an die DIN-Vorschrift 25 176, Blatt 1, »Prüfung von Holzschutzmitteln, Mykologische Kurzprüfung (Klötzchenverfahren)«, Kiefernsplintholzklötzchen mit den Abmessungen 50 x 25 x 15 mm mit jeweils 200 g/cm$^2$ Holzoberfläche der öligen Holzschutzmittelzubereitungen bestrichen. Nach vierwöchiger Lagerung wurden die behandelten Klötzchen zusammen mit den unbehandelten in Glasschalen gelegt, die als Prüfpilz Coniophora cerebella bzw. Trametes versicolor auf einem Nähragar enthielten. Die Schalen wurden anschließend in einem Klimaraum bei einer Temperatur von 22° C und einer relativen Luftfeuchte von 70% bebrütet. Nach dreimonatiger Versuchsdauer wurden die Klötzchen von anhaftendem Pilzmycel befreit und getrocknet. Anschließend wurde entsprechend dem in der DIN-Vorschrift angegebenen Bewertungsschema das Ausmaß der Holzzerstörung festgestellt.

| Behandlung des Holzes mit Zubereitungen, die 2% Wirkstoff enthalten | Ausmaß des Pilzangriffes nach dreimonatiger Versuchsdauer | |
|---|---|---|
| | Coniophora cerebella | Trametes versicolor |
| Wirkstoff 2 | 1 | 1 |
| Wirkstoff 8 | 1 | 1 |
| Wirkstoff 10 | 1 | 1 |
| Kontrolle (nur Lösungsmittel, ohne Wirkstoff) | 3a/4b | 3b |

Bewertungsschema:

1 unversehrt

2a stellenweise wenig angegriffen

2b im ganzen wenig angegriffen

3a stellenweise stark angegriffen

3b im ganzen stark angegriffen

4a stellenweise völlig zerstört

4b im ganzen völlig zerstört

Beispiel 30

Bakterizide Wirksamkeit gegenüber Staphylococcus aureus und Escherichia coli

Die Abtötungswerte gegenüber Bakterien wurden wie folgt ermittelt: Zu je 5 ml einer Verdünnung der Mittel in Wasser wurden 5 ml doppeltkonzentrierter Nährbouillon in sterilen Reagenzgläsern gegeben und vermischt. Durch Zugabe von einem Tropfen 1 : 10 verdünnter 16 Stunden alter Bouillon-Kulturen der Bakterienarten Staphylococcus aureus bzw. Escherichia coli wurden dann die Röhrchen beimpft und 24 Stunden lang bei 37° C bebrütet. Nach dieser Zeit wurden Proben aus den Röhrchen auf Bakteriennährböden übertragen und diese ebenfalls 24 Stunden lang bei 37° C bebrütet. Diejenige Verdünnungsstufe, bei welcher nach dem Übertragen einer Probe auf den Nährboden keine Bakterienentwicklung mehr erfolgte, ist als Abtötungswert angegeben.

| Wirkstoff | Bakterizide Wirksamkeit im Verdünnungsverhältnis ... | |
|---|---|---|
| | Staphylococcus aureus | Escherichia coli |
| 1 | 1: 16 000 | 1:10 000 |
| 3 | 1: 40 000 | 1:20 000 |
| 7 | 1: 40 000 | 1:20 000 |
| 8 | 1:100 000 | 1:16 000 |
| 10 | 1: 40 000 | 1:20 000 |
| 13 (bekannt) | 1: 16 000 | 1: 8 000 |

## Beispiel 31

### Fungizide Wirksamkeit gegen Plasmopara viticola an Reben

Blätter von Topfreben der Sorte Müller-Thurgau werden mit wäßrigen Suspensionen, die 80% (Gewichtsprozent) des zu prüfenden Wirkstoffes und 20% Natriumligninsulfonat in der Trockensubstanz enthalten, besprüht. Es werden 0,05 und 0,025%ige Spritzbrühe (bezogen auf die Trockensubstanz) verwendet. Nach dem Antrocknen des Spritzbelages werden die Blätter mit einer Zoosporenaufschwemmung von Plasmopara viticola infiziert. Die Pflanzen kommen dann zuerst für 16 Stunden in eine wasserdampfgesättigte (feuchte) Kammer bei 20°C und anschließend 8 Tage in ein Gewächshaus mit Temperaturen zwischen 20 und 30°C. Nach dieser Zeit werden die Pflanzen zur Beschleunigung und Verstärkung des Sporangienträgerausbruches abermals während 16 Stunden in der feuchten Kammer aufgestellt. Dann wird eine Beurteilung des Krankheitsausbruches vorgenommen; hierbei bedeuten 0 = kein Pilzbefall, abgestuft bis 5 = Totalbefall (Kontrolle).

| Wirkstoff | Befall der Blätter nach Spritzung mit + %iger Wirkstoffbrühe | |
|---|---|---|
| | + =0,05 | 0,025 |
| 1 | 0 | 0 |
| 2 | 0 | 0 |
| 3 | 0 | 3 |
| 6 | 0 | 0 |
| 7 | 0 | 0 |
| 9 | 0 | 0 |
| 10 | 0 | 0 |
| 11 (bekannt) | 1 | 3 |
| 12 (bekannt) | 2 | 4 |
| Kontrolle (unbehandelt) | 5 | 5 |

**Patentansprüche**

1. 4-Nitro-2-trichlormethylphenyldisulfide der Formel 1

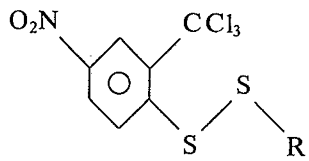

(1)

in der R einen gegebenenfalls substituierten aliphatischen Rest mit 1 bis 16 Kohlenstoffatomen, einen gegebenenfalls substituierten araliphatischen Rest mit insgesamt 7 bis 9 Kohlenstoffatomen, einen gegebenenfalls substituierten ein- oder mehrkernigen aromatischen Rest oder einen gegebenenfalls substituierten 1 bis 3 Heteroatome enthaltenden 5- oder 6gliedrigen Ring mit Sauerstoff, Stickstoff oder Schwefel als Heteroatome oder dessen Benzhomologes, den 1,2,3,4-Tetrazolyl- oder den 1-Phenyl-(1,2,3,4-tetrazolyl)-rest bedeutet.

2. Verfahren zur Herstellung der Verbindungen der Formel 1, dadurch gekennzeichnet, daß man 4-Nitro-2-trichlormethylbenzolsulfensäurechlorid mit einer eine Mercaptogruppe enthaltende Verbindung der Formel R—SH, in der R die für Formel 1 angegebenen Bedeutungen hat, in einem inerten Lösungsmittel bei Temperaturen von −40 bis 120°C umsetzt.

3. 2-(4-Nitro-2-trichlormethylphenyl-dithio)-1,3-benzthiazol.

4. Verbindungen der Formel 1, in der R einen aliphatischen Rest mit 2 bis 12 Kohlenstoffatomen bedeutet.

5. Verbindungen der Formel 1, in der R den Phenyl-Rest bedeutet.

6. Verbindungen der Formel 1, in der

R den Rest 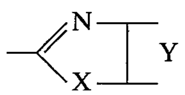

X = O, S oder NH
Y = 2 Wasserstoffatome, eine Doppelbindung oder einen anellierten Phenylrest

bedeuten.

7. Butyldithio-4-nitro-2-trichlormethylbenzol.
8. i-Propyldithio-4-nitro-2-trichlormethylbenzol.
9. n-Propyldithio-4-nitro-2-trichlormethylbenzol.
10. 3-Chlor-2-cyanphenyldithio-4-nitro-2-trichlormethylbenzol.
11. n-Dodecyldithio-4-nitro-2-trichlormethylbenzol.
12. Carboxymethyldithio-4-nitro-2-trichlormethylbenzol.
13. Methyldithio-4-nitro-2-trichlormethylbenzol.
14. 2-(4-Nitro-2-trichlormethylphenyl-dithio-)-1-cyannaphthalin.
15. (4-Chlor-2-(2)-imidazolinylphenyl)-dithio-2-trichlormethyl-4-nitrobenzol.
16. 2-(1,3-Benzimidazol)-dithio-4-nitro-2-trichlormethylbenzol.
17. [2-(5-Methylimidazolin)-4-nitro-phenyl-]-dithio-4-nitro-2-trichlormethylbenzolhydrochlorid.
18. [2-(Tetrahydropyrimidinyl-)-4-chlor-phenyl]-dithio-4-nitro-2-trichlormethylbenzol.
19. 1-Phenyl-(1,2,3,4-tetrazol)-dithio-4-nitro-2-trichlormethylbenzol.
20. 1-Acetyl-dithio-4-nitro-2-trichlormethylbenzol.
21. 2-(1,3-Thiazolin)-dithio-4-nitro-2-trichlormethylbenzol.
22. 2-Aminoäthyl-dithio-4-nitro-2-trichlormethylbenzolhydrochlorid.
23. 2-(1,3-Imidazolin)-dithio-4-nitro-2-trichlormethylbenzolhydrochlorid.
24. 3′-L-Alanin-dithio-4-nitro-2-trichlormethylbenzol.
25. 2-Pyridinyl-dithio-4-nitro-2-trichlormethylbenzol.
26. 2-Carboxyäthyl-dithio-4-nitro-2-trichlormethylbenzol.
27. Carbomethoxymethyl-dithio-4-nitro-2-trichlormethylbenzol.
28. 2-Hydroxyäthyl-dithio-4-nitro-2-trichlormethylbenzol.
29. 1-(1,2-Dicarboxyäthyl-)dithio-4-nitro-2-trichlormethylbenzol.
30. 2-(Imidazolin-2-yl)-phenyl-dithio-2-trichlormethyl-4-nitro-benzyl-hydrochlorid.
31. Mikrozid, enthaltend eine Verbindung der Formel 1 als Wirkstoff.
32. Holzschutzmittel, enthaltend 2-(4-Nitro-2-trichlormethylphenyl-dithio)-1,3-benzthiazol als Wirkstoff.
33. Die Verwendung von Verbindungen der Formel 1 und von Zubereitungen, die Verbindungen der Formel 1 als Wirkstoffe enthalten, gegen Mikroorganismen.

**Claims**

1. A 4-nitro-2-trichloromethylphenyl disulfide of the formula 1

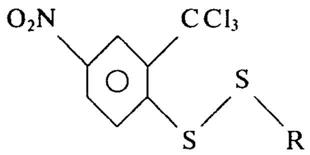

(1)

where R denotes a substituted or unsubstituted aliphatic radical of 1 to 16 carbon atoms, a substituted or unsubstituted araliphatic radical of a total of 7 to 9 carbon atoms, a substituted or unsubstituted mono- or polynuclear aromatic radical, or a substituted or unsubstituted 5- or 6-membered ring containing 1 to 3 oxygen, nitrogen or sulfur atoms as hetero atoms, or its benz homolog, 1,2,3,4-tetrazolyl or 1-phenyl-(1,2,3,4-tetrazolyl).

2. A process for the manufacture of compounds of the formula 1, characterized in that 4-nitro-2-trichloromethylbenzenesulfenyl chloride is reacted with a compound, containing a mercapto group, of the formula R—SH, where R has the meanings given for formula 1, in an inert solvent at from −40° to +120°C.

3. 2-(4-nitro-2-trichloromethylphenyldithio)-1,3-benzthiazole.

4. A compound of the formula 1, where R denotes an aliphatic radical of from 2 to 12 carbon atoms.

5. A compound of the formula 1, where R denotes phenyl.

6. A compound of the formula 1, where R denotes

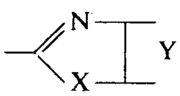

X denoting O, S or NH and Y denoting 2 hydrogen atoms, a double bond, or fused phenyl.

7. Butyldithio-4-nitro-2-trichloromethylbenzene.
8. Isopropyldithio-4-nitro-2-trichloromethylbenzene.
9. n-Propyldithio-4-nitro-2-trichloromethylbenzene.
10. 3-Chloro-2-cyanophenyldithio-4-nitro-2-trichloromethylbenzene.
11. n-Dodecyldithio-4-nitro-2-trichloromethylbenzene.
12. Carboxymethyldithio-4-nitro-2-trichloromethylbenzene.
13. Methyldithio-4-nitro-2-trichloromethylbenzene.
14. 2-(4-Nitro-2-trichloromethylphenyldithio)-1-cyanonaphthylene.
15. (4-Chloro-2-(2)-imidazolinylphenyl)-dithio-2-trichloromethyl-4-nitrobenzene.
16. 2-(1,3-Benzimidazole)-dithio-4-nitro-2-trichloromethylbenzene.
17. [2-(5-Methylimidazoline)-4-nitrophenyl]-dithio-4-nitro-2-trichloromethylbenzene hydrochloride.
18. [2-(Tetrahydropyrimidinyl)-4-chlorophenyl]-dithio-4-nitro-2-trichloromethylbenzene.
19. 1-Phenyl-(1,2,3,4-tetrazole)-dithio-4-nitro-2-trichloromethylbenzene.
20. 1-Acetyldithio-4-nitro-2-trichloromethylbenzene.
21. 2-(1,3-Thiazoline)-dithio-4-nitro-2-trichloromethylbenzene.
22. 2-Aminoethyl-dithio-4-nitro-2-trichloromethylbenzene hydrochloride.
23. 2-(1,3-Imidazoline)-dithio-4-nitro-2-trichloromethylbenzene.
24. 3′-L-Alanine-dithio-4-nitro-2-trichloromethylbenzene.
25. 2-Pyridinyldithio-4-nitro-2-trichloromethylbenzene.
26. 2-Carboxyethyldithio-4-nitro-2-trichloromethylbenzene.
27. Carbomethoxymethyldithio-4-nitro-2-trichloromethylbenzene.
28. 2-Hydroxyethyldithio-4-nitro-2-trichloromethylbenzene.
29. 1-(1,2-Dicarboxyethyl)-dithio-4-nitro-2-trichloromethylbenzene.
30. 2-(Imidazolin-2-yl)-phenyldithio-2-trichloromethyl-4-nitrobenzyl hydrochloride.
31. A microbicide containing, as active ingredient, a compound of the formula 1.
32. A wood preservative containing, as active ingredient, 2-(4-nitro-2-trichloromethylphenyldithio)-1,3-benzthiazole.
33. The use of compounds of the formula 1, and of formulations containing compounds of the formula 1 as active ingredients, against microorganisms.

**Revendications**

1. Disulfures de 4-nitro-2-trichlorométhyl-phényle de la formule 1

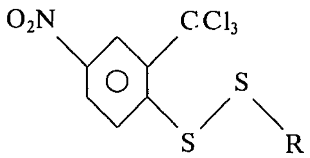

(1)

dans laquelle R désigne un reste aliphatique en $C_1$ à $C_{16}$ éventuellement substitué, un reste araliphatique éventuellement substitué avec un total de 7 à 9 atomes de carbone, un reste aromatique mono- ou polycyclique éventuellement substitué, un noyau penta- ou hexagonal éventuellement substitué et contenant 1 à 3 hétéro-atomes choisis parmi l'oxygène, l'azote et le soufre, ou ses homologues benzyliques, ou encore un reste 1,2,3,4-tétrazolyle ou 1-phényl-(1,2,3,4-tétrazolyle).

2. Procédé de préparation des composés de la formule 1, caractérisé en ce que l'on fait réagir le chlorure de l'acide 4-nitro-2-trichlorométhyl-benzène-sulfénique dans un solvant inerte, à une température entre −40 et 120°C, avec un composé contenant un groupe mercapto, de la formule R—SH, dans laquelle R possède les significations définies pour la formule 1.

3. Le 2-(4-nitro-2-trichlorométhyl-phényl-dithio)-1,3-benzothiazole.

4. Composés de la formule 1, dans laquelle R représente un reste aliphatique en $C_2$ à $C_{12}$.

5. Composés de la formule 1, dans laquelle R représente le groupe phényle.

6. Composés de la formule 1, dans laquelle R représente un reste de la formule

où X = O, S ou NH 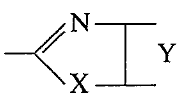

et Y = 2 atomes d'hydrogène, une double liaison ou un groupe phényle condensé.

7. Le butyl-dithio-4-nitro-2-trichlorométhyl-benzène.
8. L'isopropyl-dithio-4-nitro-2-trichloromethyl-benzène.
9. Le n-propyl-dithio-4-nitro-2-trichlorométhyl-benzène.
10. Le 3-chloro-2-cyanophényl-dithio-4-nitro-2-trichlorométhyl-benzène.
11. Le n-dodécyl-dithio-4-nitro-2-trichlorométhyl-benzène.
12. Le carboxyméthyl-dithio-4-nitro-2-trichlorométhyl-benzène.
13. Le méthyl-dithio-4-nitro-2-trichlorométhyl-benzène.
14. Le 2-(4-nitro-2-trichlorométhyl-phényl-dithio)-1-cyano-naphtalène.
15. Le (4-chloro-2-(2)-imidazolinyl-phényl)-dithio-2-trichlorométhyl-4-nitro-benzène.
16. Le 2-(1,3-benzimidazol)-dithio-4-nitro-2-trichlorométhyl-benzène.
17. Le chlorhydrate du [2-(5-méthyl-imidazoline)-4-nitro-phényl]-dithio-4-nitro-2-trichlorométhyl-benzène.
18. Le [2-(tétrahydro-pyrimidinyl)-4-chloro-phényl]-dithio-4-nitro-2-trichlorométhyl-benzène.
19. Le 1-phényl-(1,2,3,4-tétrazol)-dithio-4-nitro-2-trichlorométhyl-benzène.
20. Le 1-acétyl-dithio-4-nitro-2-trichlorométhyl-benzène.
21. Le 2-(1,3-thiazoline)-dithio-4-nitro-2-trichlorométhyl-benzène.
22. Le chlorhydrate du 2-amino-éthyl-dithio-4-nitro-2-trichlorométhyl-benzène.
23. Le chlorhydrate du 2-(1,3-imidazoline)-dithio-4-nitro-2-trichlorométhyl-benzène.
24. Le 3'-L-alanine-dithio-4-nitro-2-trichlorométhyl-benzène.
25. Le 2-pyridinyl-dithio-4-nitro-2-trichlorométhyl-benzène.
26. Le 2-carboxy-éthyl-dithio-4-nitro-2-trichlorométhyl-benzène.
27. Le carbométhoxyméthyl-dithio-4-nitro-2-trichlorométhyl-benzène.
28. Le 2-hydroxyéthyl-dithio-4-nitro-2-trichlorométhyl-benzène.
29. Le 1-(1,2-dicarboxyéthyl)-dithio-4-nitro-2-trichlorométhyl-benzène.
30. Le chlorhydrate de 2-(imidazolinyl-2)-phényl-dithio-2-trichlorométhyl-4-nitro-benzène.
31. Microbicide, contenant un composé de la formule 1 comme principe actif.
32. Produit pour la protection du bois, contenant comme principe actif le 2-(4-nitro-2-trichlorométhyl-phényl-dithio)-1,3-benzothiazole.
33. Utilisation des composés de la formule 1 et de compositions contenant des composés de la formule 1 comme principes actifs contre des micro-organismes.